(19) Europäisches Patentamt
European Patent Office
Office européen des brevets

(11) EP 0 571 832 B1

(12) **EUROPÄISCHE PATENTSCHRIFT**

(45) Veröffentlichungstag und Bekanntmachung des Hinweises auf die Patenterteilung:
**17.01.1996 Patentblatt 1996/03**

(51) Int. Cl.$^6$: **C07C 311/51**, C07C 233/91, C07C 311/48, C11D 1/02, C07C 233/11

(21) Anmeldenummer: **93107824.0**

(22) Anmeldetag: **13.05.1993**

# (54) Imide und deren Salze sowie deren Verwendung als grenzflächenaktive Wirkstoffe

Imides and their salts and their use as surface active agents

Imides et leurs sels et leur utilisation comme agents tensioactifs

(84) Benannte Vertragsstaaten:
**BE CH DE DK ES FR GB IT LI NL**

(30) Priorität: **26.05.1992 DE 4217366**

(43) Veröffentlichungstag der Anmeldung:
**01.12.1993 Patentblatt 1993/48**

(73) Patentinhaber: **BAYER AG**
**D-51368 Leverkusen (DE)**

(72) Erfinder:
- **Pohmer, Klaus, Dr.**
  **W-5000 Köln 80 (DE)**
- **Weber, Rainer, Dr.**
  **W-5068 Odenthal (DE)**
- **Dörzbach-Lange, Cornelia, Dr.**
  **W-5067 Kürten-Bechen (DE)**
- **Stachulla, Karlheinz**
  **W-5090 Leverkusen 1 (DE)**
- **Moretto, Hans-Heinrich, Dr.**
  **W-5090 Leverkusen 1 (DE)**
- **Wienand, Manfred, Dr.**
  **W-5060 Bergisch Gladbach 2 (DE)**

(56) Entgegenhaltungen:
**DE-A- 2 239 817** **US-A- 2 292 997**
**US-A- 3 041 374** **US-A- 3 637 845**
**US-A- 3 705 185** **US-A- 4 266 078**
**US-A- 4 307 170**

- **CHEMICAL ABSTRACTS, Chemical Substance Index, 11th collective index, Columbus, Ohio, US, Seite 5365**
- **CHEMICAL ABSTRACTS, Chemical Substance Index, 11th collective index, Columbus ,Ohio, US, Seite 5423**
- **CHEMICAL ABSTRACTS, Chemical Substance Index, 12th collective index, Columbus, Ohio, US, Seite 7542**
- **JOURNAL OF ORGANIC CHEMISTRY, Bd. 50, 1985, Washington, D.C., US, Seiten 4993 - 4996, H. NAGASAWA et al**
- **CHEMICAL ABSTRACTS, vol. 82, 1975, Columbus, Ohio, US; abstract no. 30924, Seite 467, Spalte 1**
- **CHEMICAL ABSTRACTS, vol. 113, 1990, Columbus, Ohio, US; abstract no. 172209, Seite 722, Spalte 1**

EP 0 571 832 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft neue Imide, deren Salze sowie deren Verwendung als grenzflächenaktive Wirkstoffe.

Perfluoralkylgruppenhaltige chemische Verbindungen finden aufgrund ihrer hohen Grenzflächenaktivität vielfältige Verwendungen in der Technik. Typische Anwendungen sind die Unterdrückung von Sprühnebeln in der Galvanik, die Verbesserung von Verlaufs- und Netzeigenschaften bei Lacken, Dispersionsklebern oder Fußbodenpflegemitteln und eine Spreitung von Wasser auf brennenden unpolaren Flüssigkeiten beim Einsatz wasserfilmbildender Feuerlöschmittel (vgl. J.N. Meußdoerffer und H. Niederprüm, Cheimkerzeitung 104 (1980) 45-52). Beispiele für solche Verbindungen sind:

$[C_8F_{17}SO_3]K$
$[C_7F_{15}COO][Na]$
$[C_8F_{17}SO_2N(C_2H_5)CH_2COO]K$
$[C_8F_{17}SO_3][N(CH_2CH_3)_4]$.

Wege zur Synthese der o.g. Verbindungen sind in "J.N. Meußdoerffer und H. Niederprüm, Chemikerzeitung 104 (1980) 45-52" ebenfalls beschrieben. Des weiteren sind gemäß der DE-A 2 239 817 Bis-perfluoralkansulfonimide der allgemeinen Formel

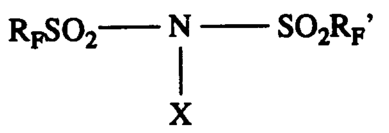

und deren Verwendung als Tenside bekannt.

Die Herstellung der perfluorierten Ausgangsverbindungen, aus denen obengenannte Verbindungen hergestellt werden, erfolgt nach drei unterschiedlichen Synthesewegen:

a) elektrochemische Fluorierung,
b) Telomerisation von Perfluorolefinen, insbesondere Tetrafluorethylen,
c) Oligomerisierung von Tetrafluorethylen.

Da die genannten Methoden zur Herstellung der perfluorierten Ausgangsverbindungen technisch sehr aufwendig sind, resultieren hohe Kosten bei der Herstellung der gewünschten perfluoralkylgruppenhaltigen chemischen Verbindungen.

Aufgabe war es, höher wirksame chemische Verbindungen zur Verfügung zu stellen, die als grenzflächenaktive Wirkstoffe eingesetzt werden können und deren Herstellung kostengünstiger ist.

Diese Aufgabe konnte durch die erfindungsgemäßen Imide und deren Salze gelöst werden.

Gegenstand der Erfindung sind fluoralkyl- und fluorarylgruppenhaltige Imide und deren Salze der allgemeinen Formel (I)

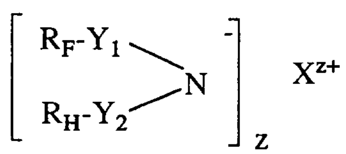

(I),

wobei

$R_F$ ein Perfluoralkylrest mit 3 bis 18 Kohlenstoffatomen, ein Perfluorarylrest mit 6 bis 12 Kohlenstoffatomen, oder ein gemischter Fluoralkylarylrest mit 7 bis 18 Kohlenstoffatomen ist,

$R_H$ ein Alkylrest mit 1 bis 30 Kohlenstoffatomen, ein Arylrest mit 6 bis 12 Kohlenstoffatomen oder ein gemischter Alkylarylrest mit 7 bis 30 Kohlenstoffatomen ist, wobei die Kohlenstoffkette der Rest auch durch Sauerstoff-, Stickstoff- oder Schwefelatome unterbrochen sein kann,

$Y_1$ und $Y_2$ jeweils eine $-SO_2$-Gruppe darstellen,

X ein Wasserstoffkation oder ein ein- oder mehrwertiges Kation ist und

z eine ganze Zahl ist, die der Ladung des Kations X entspricht.

Bevorzugt sind fluoralkyl- und fluorarylgruppenhaltige Imide und deren Salze, bei denen $R_F$ für einen Perfluoralkylrest mit 3 bis 10 Kohlenstoffatomen bzw. für einen Perfluorarylrest mit 6 bis 12 Kohlenstoffatomen steht.

Besonders bevorzugt sind Imide und deren Salze, bei denen $R_H$ für einen Alkylrest mit 6 bis 20 Kohlenstoffatomen, einen Arylrest mit 6 bis 12 Kohlenstoffatomen oder einen gemischten Alkylarylrest mit 7 bis 20 Kohlenstoffatomen steht.

Als Kation X werden vorzugsweise Alkali- oder Erdalkalikationen, Ammoniumkation oder ein- oder mehrfach alkyl- und/oder arylsubstituierte Ammoniumkationen oder -polykationen eingesetzt.

Besonders bevorzugte Imide bzw. Imidsalze weisen z.B. folgende Strukturen auf:

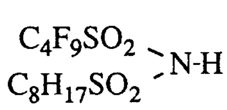

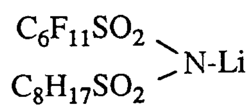

Die erfindungsgemäßen Imide und deren Salze lassen sich z.B. durch mehrstufige Umsetzung von Fluorsulfonsäuren oder deren Derivaten mit Ammoniak und Sulfonsäuren oder deren Derivaten herstellen. Im folgenden ist ein möglicher Syntheseweg beispielhaft wiedergegeben.

In der ersten Stufe erfolgt die Herstellung des Salzes des Fluorsulfonsäureamides:

$$R_F\text{-}Y_1\text{-}A_1 + NH_3 + 2N(CH_2CH_3)_3 \rightarrow [R_F\text{-}Y_1\text{-}NH][HN(CH_2CH_3)_3] + [HN(CH_2CH_3)_3]A_1,$$

wobei
$R_F$ und $Y_1$ dieselbe Bedeutung wie oben haben und

$A_1$ eine reaktive Abgangsgruppe, wie z.B. ein Halogenatom, eine Hydroxy-, eine Alkoxy- oder eine Carboxygruppe, ist.

In der zweiten Stufe erfolgt die Umsetzung des entstandenen Fluorsulfonsäureamidsalzes zum Triethylammoniumsalz des Imides:

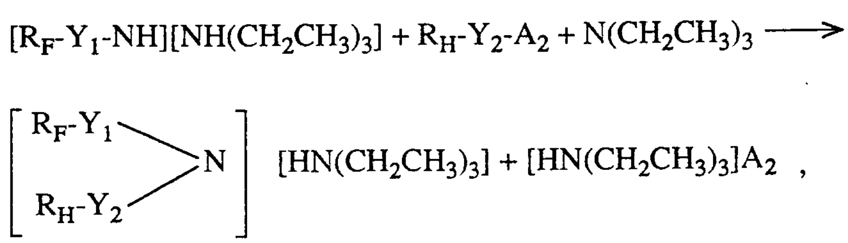

wobei $R_H$ und $Y_2$ dieselbe Bedeutung wie oben haben und

$A_2$ eine reaktive Abgangsgruppe, wie z.B. ein Halogenatom, eine Hydroxy-, eine Alkoxy- oder eine Carboxygruppe, ist.

In einer dritten Stufe kann das Triethylammoniumsalz mit Schwefelsäure umgesetzt werden, wodurch das freie Imid entsteht:

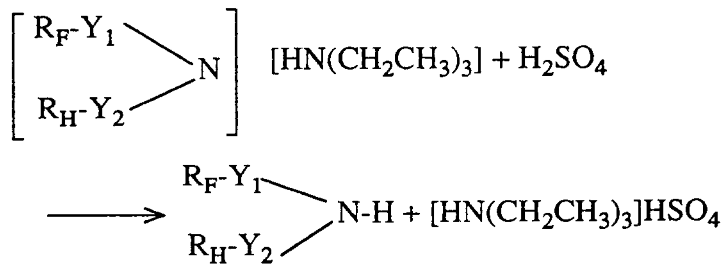

oder mit einer Base in ein beliebiges Salz umgewandelt werden:

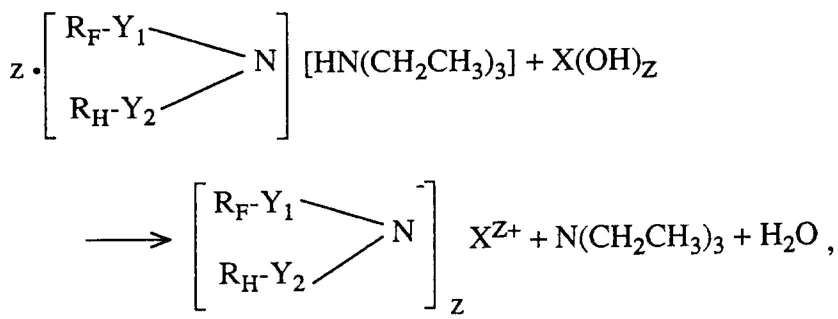

wobei X und z dieselbe Bedeutung wie oben haben.

Für das vorgenannte mehrstufige Verfahren können folgende Ausgangsverbindungen eingesetzt werden:

Beispiele für Fluorsulfonsäuren:

| | |
|---|---|
| Perfluorbutansulfonsäure | $CF_3$-$(CF_2)_3$-$SO_3H$ |
| Perfluorhexansulfonsäure | $CF_3$-$(CF_2)_5$-$SO_3H$ |
| Perfluoroctansulfonsäure | $CF_3$-$(CF_2)_7$-$SO_3H$ |
| Perfluorbenzolsulfonsäure | $C_6F_5$-$SO_3H$ |
| Perfluortoluolsulfonsäure | $CF_3$-$C_6F_4$-$SO_3H$ |

Beispiele für Fluorsulfonsäurederivate:

| | |
|---|---|
| Perfluorbutansulfonsäurefluorid | $CF_3(CF_2)_3SO_2F$ |
| Perfluorhexansulfonsäurefluorid | $CF_3(CF_2)_5SO_2F$ |
| Perfluoroctansulfonsäure | $CF_3(CF_2)_7SO_2F$ |
| Perfluorbenzolsulfonsäurechlorid | $C_6F_5SO_2Cl$ |

Beispiele für Sulfonsäuren:

| | |
|---|---|
| Methansulfonsäure | $CH_3SO_3H$ |
| Ethansulfonsäure | $CH_3CH_2SO_3H$ |
| Propansulfonsäure | $CH_3(CH_2)_2SO_3H$ |
| Butansulfonsäure | $CH_3(CH_2)_3SO_3H$ |
| Pentansulfonsäure | $CH_3(CH_2)_4SO_3H$ |
| Hexansulfonsäure | $CH_3(CH_2)_5SO_3H$ |
| Vinylsulfonsäure | $CH_2$=$CHSO_3H$ |
| Methallylsulfonsäure | $CH_2$=$C(CH_3)$-$CH_2$-$SO_3H$ |

Benzolsulfonsäure $C_6H_5SO_3H$
Toluolsulfonsäure $CH_3C_6H_4SO_3H$

Beispiele für Sulfonsäurederivate:

Sulfonsäurehalogenide
Sulfonsäureester
Sulfonsäureanhydride
Sulfonsäuresalze.

Bei den erfindungsgemäßen Imidsalzen steht X vorzugsweise für ein Kation aus der Reihe der Alkali- oder Erdalkalikationen, für ein Ammoniumkation oder ein ein- oder mehrfach alkyl- und/oder arylsubstituiertes Ammoniumkation. Beispiele für solche Kationen sind: $Na^+$, $Li^+$, $K^+$, $Ca^{2+}$, $Mg^{2+}$, Tetraethylammoniumkation.

Ein weiterer Gegenstand der Erfindung ist die Verwendung der erfindungsgemäßen Imide und ihrer Salze als grenzflächenaktive Wirkstoffe.

Aufgrund der sehr hohen Grenzflächenaktivität der erfindungsgemäßen Imide und ihrer Salze, die teilweise höher liegt als die von bekannten perfluorgruppenhaltigen chemischen Verbindungen, wirken die erfindungsgemäßen Imide und ihre Salze als hochwirksame Tenside, deren Entfaltung der Wirksamkeit schneller als bei den bekannten perfluoralkylgruppenhaltigen Verbindungen eintritt, wodurch sie beispielsweise in den folgenden Anwendungsgebieten eingesetzt werden können:

In elektrolytischen Prozessen (z.B. bei der galvanischen Verchromung, Verkupferung und Vernickelung, beim Anodisieren und bei der elektrolytischen Entfettung) können die erfindungsgemäßen Verbindungen zur Sprühnebelunterdrückung und zur Verhinderung von Austragsverlusten zugesetzt werden.

In nicht elektrolytischen Badprozessen (z.B. bei der chemischen Verkupferung oder Vernickelung, bei der chemischen Entfettung oder Entrostung, beim Ätzen oder Gravieren, beim Glanztauchen, beim Beizen, Brünieren oder Passivieren, beim Eloxieren oder beim Entmetallisieren) können die erfindungsgemäßen Verbindungen als Sprühnebelunterdrücker und Reinigungshilfsmittel zugesetzt werden.

In Reinigungs- und Pflegemitteln (wie z.B. in Glas-, Herd-, Auto-, Gebäude-, Fassaden- oder Metalloberflächenreinigungsmitteln, in Fleckentfernern, in Shampoos, in Polituren für Möbel, Autos usw., in Selbstglanzemulsionen oder in Wachsen) können die erfindungsgemäßen Verbindungen als Verlaufs-, Spreit- und Netzmittel sowie zur Unterstützung der Eigenschaften, die die Wiederanschmutzung verhindern, zugesetzt werden.

Die erfindungsgemäßen Verbindungen können als solche oder in Formulierungen als Antibeschlagmittel oder Anlaufschutz (z.B. für Gläser, Metalle oder Kunststoffe) eingesetzt werden.

Die erfindungsgemäßen Verbindungen können als solche oder in Formulierungen als Korrosionsinhibitoren oder Korrosionsschutzüberzüge (z.B. in Polymerisationsreaktionen, für Füllstoffe, Fasern, Salze oder magnetische Feststoffe, in Lacken oder Blutersatzstoffen) eingesetzt werden.

Aufgrund ihrer Neigung, gasdichte Sperrschichten auszubilden und damit das Verdampfen oder Verdunsten von Flüssigkeiten zu verhindern, eignen sich die erfindungsgemäßen Verbindungen auch als Zusätze zu Feuerlöschmitteln.

Die erfindungsgemäßen Verbindungen können als Formtrennmittel eingesetzt werden.

In Farben und Lacken bewirkt ein Zusatz der erfindungsgemäßen Verbindungen eine Verbesserung der Verlaufs-, Netz- und Haftungseigenschaften. Außerdem verhindern sie durch Förderung der Entlüftung die Bildung von Oberflächendefekten (wie z.B. Kraterbildung oder Kantenflucht). Ferner wird durch ihren Zusatz eine bessere Verteilung der Pigmente erreicht. Von besonderem Vorteil ist die nicht schaumstabilisierende Wirkung der erfindungsgemäßen Verbindungen in Rezepturen bei der Herstellung von wasserverdünnbaren Lacken.

Die Neigung der erfindungsgemäßen Verbindungen, hydrophobe und oleophobe Sperrschichten auszubilden, macht einen Einsatz in Bautenschutzmitteln (z.B. zur Isolierung gegen Umwelteinflüsse) möglich.

Die erfindungsgemäßen Verbindungen können als Fließ- oder Gleitmittel (z.B. in Mineralerzen oder -salzen, auf Magnetbändern oder in Baustoffen) eingesetzt werden.

Die erfindungsgemäßen Verbindungen eignen sich als Schmiermittel, Schneidölzusätze oder Hydrauliköle.

Die erfindungsgemäßen Verbindungen können als Bohrhilfsmittel (z.B. Leistungssteigerung bei Ölbohrungen) eingesetzt werden.

In Fotochemikalien oder bei der Filmherstellung können die erfindungsgemäßen Verbindungen (z.B. als Netzmittel oder Antistatikum) eingesetzt werden.

In Pflanzenschutzmitteln können die erfindungsgemäßen Verbindungen (z.B. als Netz- und Verlaufsmittel) eingesetzt werden.

Ein Zusatz der erfindungsgemäßen Verbindungen zu Ausrüstungsmitteln für Textilien, Leder oder Papier kann beispielsweise die Benetzung oder das Eindringen des Ausrüstungsmittels fördern, zu einer Entschäumung führen oder dessen Hydrophob-/Oleophobwirkung unterstützen.

Die erfindungsgemäßen Verbindungen können als Flammschutzmittel (z.B. in Kunststoffen) eingesetzt werden.

Außerdem ist ein Einsatz der erfindungsgemäßen Verbindungen als Flüssigkristalle möglich.

Aufgrund ihrer Säurestärke ist ein Einsatz der erfindungsgemäßen Verbindungen als Katalysator (z.B. bei Verseifungs- oder Sulfonierungsreaktionen oder bei Polymerisationsreaktionen) möglich.

Die Erfindung soll anhand folgender Beispiele näher erläutert werden.

Beispiel 1

In einer Glaskolben-Rührapparatur werden bei Raumtemperatur unter exothermer Reaktion 0,20 Mol (120 g) Triethylammoniumperfluoroctylsulfonamid und 0,20 Mol (20,2 g) Triethylamin umgesetzt. Der Ansatz wird bis zum Rückfluß erhitzt, 0,2 Mol (39,2 g) Octylsulfonylfluorid zudosiert und anschließend 1 h nachgerührt. Nach Abkühlen auf ca. 50°C wird mit 20 %iger HCl auf pH=6 eingestellt.

Nach mehrmaligem Waschen mit Wasser wird die isolierte Produktphase bei 60°C und 24 mbar getrocknet. Die Ausbeute an Triethylammonium-N-octylsulfonylperfluoroctylsulfonimid beträgt 129 g (entsprechend 83 % der Theorie).

Beispiel 2

In einer Glaskolben-Rührapparatur werden bei Raumtemperatur 0,1 Mol (77,6 g) Triethylammonium-N-octylsulfonylperfluoroctylsulfonimid mit 0,1 Mol (4,2 g) Lithiumhydroxid-1-hydrat, gelöst in 30 g Wasser, versetzt. Anschließend wird bis zum Rückfluß erhitzt.

Nach einer Rührzeit von 1 Stunde werden Wasser und Triethylamin bei 90°C abdestilliert. Das erhaltene Produkt wird bei 60°C und 24 mbar getrocknet. Die Ausbeute des Lithiumsalzes von N-Octylsulfonyl-perfluoroctylsulfonimid beträgt 67,5 g (entsprechend 100 % der Theorie).

Beispiel 3

In einer Glaskolben-Rührapparatur werden bei Raumtemperatur unter exothermer Reaktion 0,1 Mol (60 g) Triethylammonium-perfluoroctylsulfonamid und 0,1 Mol (10 g) Triethylamin in 150 ml Diisopropylether umgesetzt. Der Ansatz wird bis zum Rückfluß erhitzt, 0,1 Mol (19,1 g) Tosylchlorid zudosiert und anschließend 1 h nachgerührt. Nach Abkühlen auf ca. 50°C wird mit 20 %iger HCl auf pH=6 eingestellt.

Die etherische Phase wird mehrmals mit Wasser gewaschen. Anschließend wird der Ether bei 20°C und 24 mbar abdestilliert. Die Ausbeute an Triethylammonium-N-tosyl-perfluoroctylsulfonimid beträgt 20 g (entsprechend 26,5 % der Theorie).

Beispiel 4

In einer Glaskolben-Rührapparatur werden bei Raumtemperatur unter exothermer Reaktion 0,1 Mol (40 g) Triethylammonium-perfluorbutylsulfonamid und 0,1 Mol (10 g) Triethylamin in 150 ml Diisopropylether umgesetzt. Der Ansatz wird bis zum Rückfluß erhitzt, 0,1 Mol (19,1 g) Tosylchlorid zudosiert und anschließend 1 h nachgerührt. Nach Abkühlen auf ca. 50°C wird mit 20 %iger HCl auf pH = 6 eingestellt.

Die etherische Phase wird mehrmals mit Wasser gewaschen. Anschließend wird der Ether bei 60°C und 24 mbar abdestilliert. Die Ausbeute an Triethylammonium-N-tosyl-perfluorbutylsulfonimid beträgt 20 g (entsprechend 36,1 % der Theorie).

Beispiel 5

In einer Glaskolben-Rührapparatur werden bei Raumtemperatur unter exothermer Reaktion 0,25 Mol (100 g) Triethylammoniumperfluorbutylsulfonamid und 0,25 Mol (25 g) Triethylamin umgesetzt. Der Ansatz wird bis zum Rückfluß erhitzt, 0,25 Mol (49 g) Octylsulfonylfluorid zudosiert und anschließend 1 h nachgerührt. Nach Abkühlen auf ca. 50°C wird mit 20 %iger HCl auf pH = 6 eingestellt.

Nach mehrmaligem Waschen mit Wasser wird die Produktphase bei 60°C und 24 mbar getrocknet. Die Auswaage an Triethylammonium-N-octylsulfonyl-perfluorbutylsulfonimid beträgt 135,9 g (entsprechend 94,4 % der Theorie).

Beispiel 6

In einer Glaskolben-Rührapparatur werden bei Raumtemperatur 0,236 Mol (135,9 g) Triethylammonium-N-octylsulfonyl-perfluorbutylsulfonimid mit 0,236 Mol (9,4 g) Natriumhydroxid, gelöst in 60 g Wasser, versetzt. Anschließend wird bis zum Rückfluß erhitzt. Nach einer Rührzeit von 1 Stunde werden Wasser und Triethylamin bei 90°C abdestilliert. Das erhaltene Produkt wird bei 60°C und 24 mbar getrocknet. Die Ausbeute des Natriumsalzes von N-Octylsulfonyl-perfluorbutylsulfonimid beträgt 117,3 g (entsprechend 100 % der Theorie).

Beispiel 7

In einer Glaskolben-Rührapparatur werden bei Raumtemperatur 0,236 Mol (135,9 g) Triethylammonium-N-octylsulfonyl-perfluorbutylsulfonimid mit 0,236 Mol (13,2 g) Kaliumhydroxid, versetzt in 60 g Wasser, versetzt. Anschließend wird bis zum Rückfluß erhitzt. Nach einer Rührzeit von 1 Stunde werden Wasser und Triethylamin bei 90°C abdestilliert. Das erhaltene Produkt wird bei 60°C und 24 mbar getrocknet. Die Ausbeute des Kaliumsalzes von N-Octylsulfonyl-perfluorbutylsulfonimid beträgt 121,0 g (entsprechend 100 % der Theorie).

Beispiel 8

In einer Glaskolben-Rührapparatur werden bei Raumtemperatur 0,236 Mol (135,9 g) Triethylammonium-N-octylsulfonyl-perfluorbutylsulfonimid mit 0,236 Mol (9,9 g) Lithiumhydroxid-1-hydrat, gelöst in 60 g Wasser, versetzt. Anschließend wird bis zum Rückfluß erhitzt. Nach einer Rührzeit von 1 Stunde werden Wasser und Triethylamin bei 90°C abdestilliert. Das erhaltene Produkt wird bei 60°C und 24 mbar getrocknet. Die Ausbeute des Lithiumsalzes von N-Octysulfonyl-perfluorbutylsulfonimid beträgt 113,5 g (entsprechend 100 % der Theorie).

Beispiel 9

In diesem Beispiel wird das erfindungsgemäße Lithium-N-octylsulfonyl-perfluorbutylsulfonimid im Vergleich zu zwei bekannten Perfluortensiden (Lithium-bis-perfluorbutylsulfonimid und Tetraethylammonium-perfluoroctylsulfonat) auf ihre grenzflächenaktive Wirksamkeit untersucht. Dazu wird die Oberflächenspannung in Abhängigkeit von der Tensidkonzentration in Wasser bei 20°C mit einem Tensiometer (Typ TE 1C der Firma Lauda) gemessen. Die Ergebnisse sind in Abbildung 1 dargestellt.

Gemäß Abbildung 1 wird mit der erfindungsgemäßen Verbindung bereits bei einer deutlich geringeren Einsatzkonzentration als bei den bekannten Verbindungen eine Oberflächenspannung von ca. 20 mN/m erreicht.

**Patentansprüche**

1. Fluoralkyl- und fluorarylgruppenhaltige Imide und deren Salze der allgemeinen Formel (I)

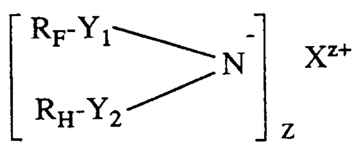

(I),

wobei

$R_F$ ein Perfluoralkylrest mit 3 bis 18 Kohlenstoffatomen, ein Perfluorarylrest mit 6 bis 12 Kohlenstoffatomen oder ein gemischter Fluoralkylarylrest mit 7 bis 18 Kohlenstoffatomen ist,

$R_H$ ein Alkylrest mit 1 bis 30 Kohlenstoffatomen, ein Arylrest mit 6 bis 12 Kohlenstoffatomen oder ein gemischter Alkylarylrest mit 7 bis 30 Kohlenstoffatomen ist, wobei die Kohlenstoffkette der Rest auch durch Sauerstoff-, Stickstoff- oder Schwefelatome unterbrochen sein kann,

$Y_1$ und $Y_2$ jeweils eine -$SO_2$-Gruppe darstellen,

X ein Wasserstoffkation oder ein ein- oder mehrwertiges Kation ist und

z eine ganze Zahl von 1 bis 7 ist, die der Ladung des Kations X entspricht.

2. Imide und deren Salze gemäß Anspruch 1, dadurch gekennzeichnet, daß $R_F$ ein Perfluoralkylrest mit 3 bis 10 Kohlenstoffatomen oder ein Perfluorarylrest mit 6 bis 12 Kohlenstoffatomen ist.

3. Imide und deren Salze gemäß Anspruch 1 oder 2, dadurch gekennzeichnet, daß $R_H$ ein Alkylrest mit 6 bis 20 Kohlenstoffatomen, ein Arylrest mit 6 bis 12 Kohlenstoffatomen oder ein gemischter Alkylarylrest mit 7 bis 20 Kohlenstoffatomen ist.

4. Imidsalze gemäß einem oder mehreren der Ansprüche 1 bis 3, dadurch gekennzeichnet, daß X ein Kation aus der Reihe der Alkali- oder Erdalkalikationen, ein Ammoniumkation oder ein ein- oder mehrfach alkyl- und/oder arylsubstituiertes Ammoniumkation ist.

5. Verwendung der fluoraryl- und fluoralkylgruppenhaltigen Imide und deren Salze gemäß den Ansprüchen 1 bis 4 als grenzflächenaktive Wirkstoffe.

**Claims**

1. Fluoroalkyl- and fluoroaryl-group-containing imides and their salts, of general formula (I)

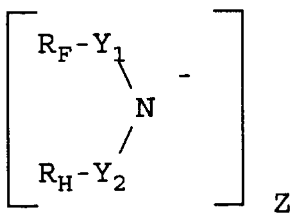

$X^{z+}$ (I),

wherein

$R_F$ is a perfluoroalkyl group with 3 to 18 carbon atoms, a perfluoroaryl group with 6 to 12 carbon atoms or a mixed fluoroalkylaryl group with 7 to 18 carbon atoms,

$R_H$ is an alkyl group with 1 to 30 carbon atoms, an aryl group with 6 to 12 carbon atoms or a mixed alkylaryl group with 7 to 30 carbon atoms, wherein the carbon chain of the group can also be interrupted by oxygen, nitrogen or sulphur atoms,

$Y_1$ and $Y_2$ each represents an $SO_2$ group,

X is a hydrogen cation or a uni- or multivalent cation and

z is a whole number from 1 to 7, corresponding to the charge of the cation X.

2. Imides and their salts according to Claim 1, characterized in that $R_F$ is a perfluoroalkyl group with 3 to 10 carbon atoms or a perfluoroaryl group with 6 to 12 carbon atoms.

3. Imides and their salts according to Claim 1 or 2, characterized in that $R_H$ is an alkyl group with 6 to 20 carbon atoms, an aryl group with 6 to 12 carbon atoms or a mixed alkylaryl group with 7 to 20 carbon atoms.

4. Imide salts according to one or more of Claims 1 to 3, characterized in that X is a cation from the series of alkali metal or alkaline earth metal cations, an ammonium cation or a mono- or polyalkyl- and/or -aryl-substituted ammonium cation.

5. The use of the fluoroaryl- and fluoroalkyl-group-containing imides and their salts according to Claims 1 to 4 as surfactant agents.

## Revendications

1. Imides à groupes fluoralkyle et fluoraryle, et leurs sels, de formule générale I

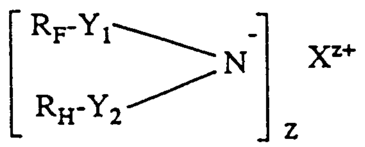

(I),

dans laquelle

$R_F$ représente un groupe perfluoralkyle contenant 3 à 18 atomes de carbone, un groupe perfluoraryle contenant 6 à 12 atomes de carbone ou un groupe fluoralkylaryle mixte contenant 7 à 18 atomes de carbone,

$R_H$ représente un groupe alkyle contenant 1 à 30 atomes de carbone, un groupe aryle contenant 6 à 12 atomes de carbone ou un groupe alkylaryle mixte contenant 7 à 30 atomes de carbone, la chaîne carbonée de ces groupes pouvant être également interrompue par des atomes d'oxygène, d'azote ou de soufre,

$Y_1$ et $Y_2$ représentent chacun un groupe $-SO_2-$,

X représente un cation d'hydrogène ou un cation mono- ou polyvalent et

z est un nombre entier allant de 1 à 7 et correspondant à la charge du cation X.

2. Imides et leurs sels selon revendication 1, caractérisés en ce que $R_F$ est un groupe perfluoralkyle en $C_3$-$C_{10}$ ou perfluoraryle en $C_6$-$C_{12}$.

3. Imides et leurs sels selon revendication 1 ou 2, caractérisés en ce que $R_H$ représente un groupe alkyle en $C_6$-$C_{20}$, un groupe aryle en $C_6$-$C_{12}$ ou un groupe alkylaryle mixte en $C_7$-$C_{20}$.

4. Sels d'imides selon une ou plusieurs des revendications 1 à 3, caractérisés en ce que X est un cation choisi parmi les cations de métaux alcalins ou alcalino-terreux, les cations ammonium ou les cations ammonium portant un ou plusieurs substituants alkyle et/ou aryle.

5. Utilisation des imides à groupes fluoraryle et fluoralkyle et de leurs sels, selon les revendications 1 à 4, en tant qu'agents tensioactifs.

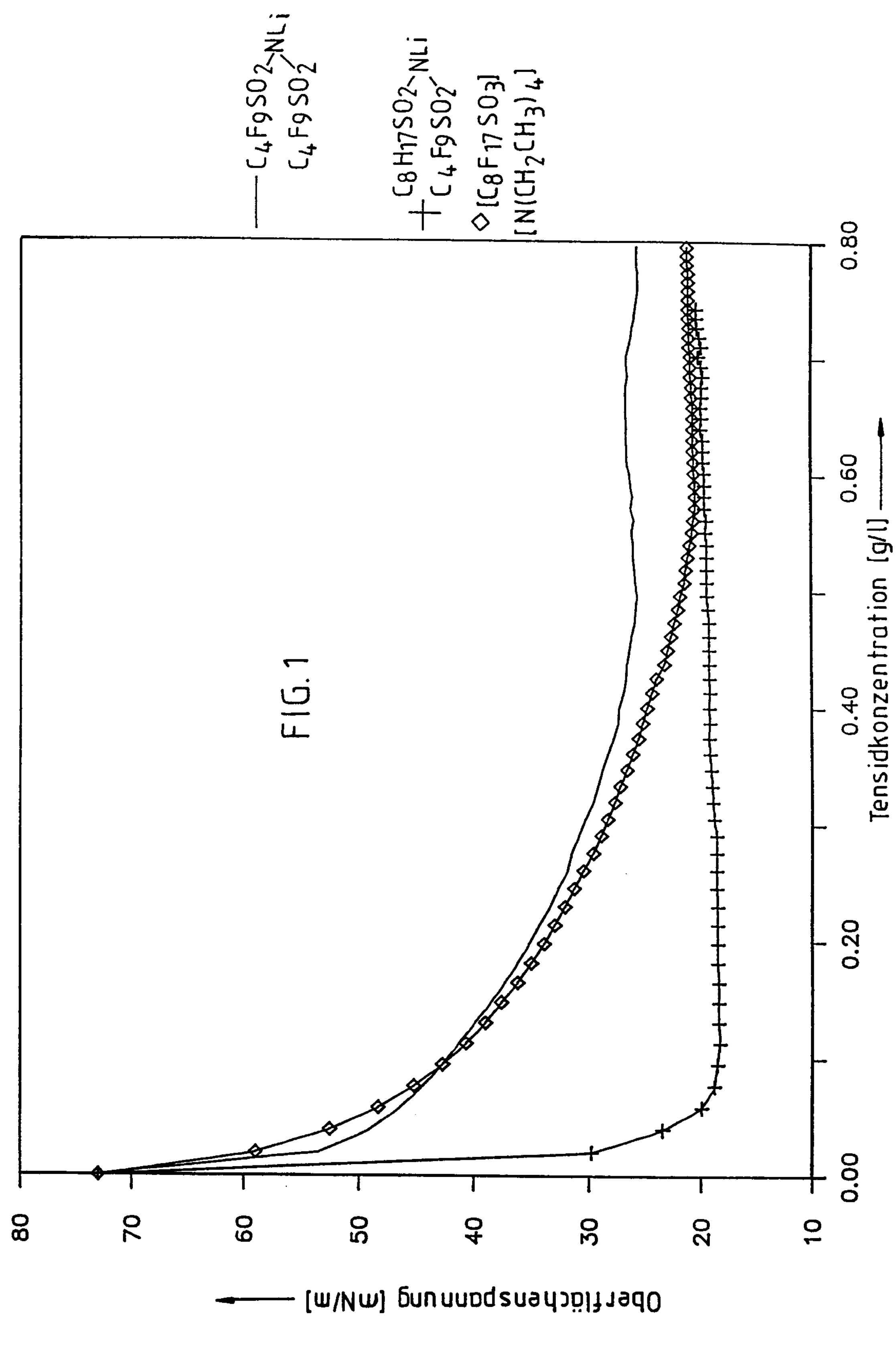

Oberflächenspannung [mN/m]
80
70
60
50
40
30
20
10
0.00
0.20
0.40
0.60
0.80
Tensidkonzentration [g/l]
$C_4F_9SO_2$-NLi
$C_4F_9SO_2$
$C_8H_{17}SO_2$-NLi
$C_4F_9SO_2$
$[C_8F_{17}SO_3]$
$[N(CH_2CH_3)_4]$


FIG.1